# FASCICULE DE BREVET EUROPEEN

(11) **EP 1 575 433 B1**
(45) Date de publication et mention de la délivrance du brevet: **05.11.2008**
(21) Numéro de dépôt: 03773815.0
(22) Date de dépôt: 02.10.2003
(51) Int. Cl.: A61B 17/70

(54) **SYSTEME DE FIXATION A PLAQUE**
FIXIERUNGSSYSTEM FÜR KNOCHENPLATTE
PLATE FIXING SYSTEM

(30) Priorité: 07.10.2002 FR 0212397; 28.02.2003 FR 0302503
(43) Date de publication de la demande: 21.09.2005
(73) Titulaire: Abbott Spine, La Cité Mondiale, 33000 Bordeaux (FR)
(72) Inventeur: BACCELLI, Christian, F-33640 AYGUEMORTE LES GRAVES (FR); MANGIONE, Paolo, F-33600 PESSAC (FR)
(74) Mandataire: Balesta, Pierre
(86) Numéro de dépôt international: PCT/FR2003/002890
(87) Numéro de publication internationale: WO 2004/032772

(56) Documents cités:
- WO-A-97/37620
- WO-A-02/054966
- FR-A- 2 812 535
- FR-A- 2 827 757

## Description

La présente invention a pour objet un système d'immobilisation d'au moins deux vertèbres l'une par rapport à l'autre.

De façon plus précise, la présente invention concerne un système mécanique qui peut être utilisé par un chirurgien pour être monté sur deux ou plus de deux vertèbres de la colonne vertébrale afin d'immobiliser relativement ces deux vertèbres.

De tels systèmes sont en soi connus. Ils sont constitués par deux vis pédiculaires qui sont vissées dans le pédicule des deux vertèbres à immobiliser et par une pièce allongée, le plus souvent appelée plaque dont les extrémités sont fixées sur les têtes des deux vis par des organes mécaniques de solidarisation de telle manière qu'ainsi, la distance entre les têtes des vis et donc la distance entre les deux vertèbres reste fixe.

Le plus souvent, les têtes de vis sont sphériques pour former avec l'organe de solidarisation correspondant un système rotulant qui permet le montage de la plaque quelle que soit la direction relative des deux vis sans introduire de contrainte sur les vis, et donc sur les vertèbres dans lesquelles celles-ci sont fixées. Un tel système est connu de la demande de brevet WO-A-02054966.

On comprend qu'une des qualités que doit présenter un tel système est que la solidarisation de la plaque et des vis soit suffisamment efficace pour qu'elle puisse absorber les efforts résultants des mouvements du patient qui porte le système d'immobilisation. D'autre part, il est bien sûr souhaitable que la mise en place de ce système soit aussi aisée que possible pour le chirurgien et que cette mise en place demande un temps d'intervention aussi réduit que possible.

Un objet de la présente invention est de fournir un système d'immobilisation d'au moins deux vertèbres du type mentionné ci-dessus qui remplisse mieux les deux conditions énoncées ci-dessus.

Pour atteindre ce but, selon l'invention, le dispositif d'immobilisation d'au moins deux vertèbres comprenant au moins deux vis, une pièce allongée de liaison et au moins deux organes de solidarisation, chaque vis comprenant un corps de vissage et une tête ayant la forme d'une partie de sphère constituée par une première portion de surface sphérique disposée entre le corps de la vis et un plan diamétral orthogonal à l'axe du corps de la vis et une deuxième portion de surface sphérique ; **caractérisé en ce que**
chaque organe de solidarisation comprend au moins :
- une pièce de serrage ; et
- une pièce de solidarisation formée d'une seule pièce ayant la forme d'une bague ayant une paroi latérale entourant un passage axial, ladite paroi comportant une première ouverture apte à recevoir ladite pièce de serrage et à coopérer avec elle et une deuxième ouverture comportant une première et une deuxième partie, lesdites deux parties communiquant entre elles et étant angulairement décalées par rapport à l'axe (Y,Y') de la pièce de solidarisation, ladite première partie présentant un axe diamétral (Z, Z') sensiblement confondu avec celui de ladite première ouverture et un rebord formant une portée pour ladite première portion de surface sphérique de la tête de vis, ladite deuxième partie de la deuxième ouverture permettant le libre passage de la tête de la vis, ledit passage axial étant apte à recevoir au moins une extrémité de ladite pièce de liaison et ladite tête de vis, par quoi, la tête de la vis peut être librement introduite dans le passage axial de la pièce de solidarisation, par ladite deuxième partie de la deuxième ouverture, par rotation de ladite pièce de solidarisation, la portée de la première partie de la deuxième ouverture est amenée en regard de la première portion de surface sphérique de la tête de vis, et, par activation de la pièce de serrage, l'extrémité de la pièce de liaison et la tête de la vis sont immobilisées en rotation et en translation par rapport à ladite pièce de solidarisation.

On comprend que, d'une part, le système de solidarisation de la tête de la vis pédiculaire et de la pièce de liaison allongée est réellement efficace. En effet, la tête de la vis pédiculaire est plaquée avec une force convenable sur la portée qui entoure la première partie de la deuxième ouverture sous l'effet de l'organe de serrage, l'axe de l'organe de serrage et l'axe de l'ouverture comportant la portée étant sensiblement confondus.

On comprend en outre qu'une pièce de solidarisation, qui est formée d'une seule pièce, confère plusieurs avantages : d'une part, cela évite au chirurgien la nécessité d'assembler un nombre important de pièces dans des conditions opératoires délicates et, d'autre part, la bague formée d'une seule pièce peut présenter un profil extérieur lisse et régulier, essentiellement exempt d'aspérités qui risqueraient d'endommager les tissus humains avoisinants.

On comprend également que la mise en place de ce système d'immobilisation est relativement aisé puisque, après le vissage des vis pédiculaires dans les pédicules des deux vertèbres à immobiliser, chaque organe de liaison peut être aisément engagé sur la tête de la vis, grâce à la deuxième partie de la deuxième ouverture et que le verrouillage de la tête de la vis, dans l'organe de solidarisation, est également aisé puisqu'il suffit de faire pivoter l'organe de solidarisation et d'activer l'organe de serrage.

Selon un mode préféré de mise en oeuvre, chaque organe de solidarisation comprend, en outre, une pièce intermédiaire insérable dans le passage axial de la pièce de solidarisation, ladite pièce intermédiaire présentant une première face apte à être mise en regard de la face interne de la paroi de la pièce de solidarisation, ladite pièce intermédiaire présentant un évidement, débouchant dans ladite première face, formant une portée pour au moins une partie de ladite deuxième portion de surface sphérique de la tête de vis, et une deuxième face d'appui apte à coopérer avec l'extrémité de la pièce de liaison, par quoi, lorsque ladite pièce intermédiaire est insérée dans le passage axial de la pièce de liaison, la force de serrage développée par l'organe de serrage est transmise à ladite pièce intermédiaire par l'intermédiaire de l'extrémité de la pièce de liaison.

On comprend que dans ce mode de réalisation préférentiel, le serrage de la tête de la vis sur la portée d'appui est réalisé par l'organe de serrage via la pièce intermédiaire. La présence de cette pièce intermédiaire permet d'améliorer la qualité du contact entre cette pièce intermédiaire qui reçoit la force de serrage développée par l'organe de serrage et la tête sphérique de la vis pédiculaire. Selon un mode préféré de réalisation de cette variante, la pièce intermédiaire peut être pré-montée dans le passage axial de la pièce de solidarisation avant son utilisation. La présence de cette pièce ne rend donc pas plus complexe l'utilisation du système d'immobilisation relative des vertèbres.

D'autres caractéristiques et avantages de l'invention apparaîtront mieux à la lecture de la description qui suit de plusieurs modes de réalisation de l'invention donnés à titre d'exemple non limitatif. La description se réfère aux figures annexées sur lesquelles :
la figure 1 est une vue en perspective montrant l'implantation du système d'immobilisation sur les vertèbres ;
la figure 2 est une vue éclatée montrant les différents composants du système d'immobilisation ;
la figure 3 est une vue en perspective de la pièce de solidarisation ;
la figure 4A est une vue de dessus de la pièce de solidarisation ;
la figure 4B est une vue en coupe selon la ligne B-B de la figure 4A ;
la figure 4C est une vue en coupe selon la ligne C-C de la figure 4B ;
la figure 5 est une vue en coupe verticale montrant la solidarisation d'une vis et de la pièce de liaison, selon un premier mode de mise en oeuvre de l'invention ;
la figure 6 est une vue en perspective de la pièce intermédiaire selon une première variante de réalisation ;
la figure 7A est une vue de dessus de la pièce intermédiaire de la figure 6 ;
la figure 7B est une vue en coupe selon la ligne B-B de la figure 7A ;
la figure 7C est une vue en coupe selon la ligne C-C de la figure 7A ;
la figure 8A est une vue en coupe longitudinale de la pièce de liaison selon une première variante de réalisation ;
la figure 8B est une vue de dessous de la pièce de liaison de la figure 8A ;
la figure 8C est une vue en coupe selon la ligne C-C de la figure 8A ;
les figures 9A, 9B et 9C illustrent le mode de solidarisation d'une vis avec la pièce de liaison, selon le mode perfectionné de mise en oeuvre de l'invention ;
la figure 10 est une vue en perspective d'une deuxième variante de réalisation du système de solidarisation ;
la figure 11A est une vue en perspective d'une variante de réalisation de la pièce intermédiaire ;
la figure 11B est une vue de dessus de la pièce intermédiaire de la figure 11A ;
la figure 11C est une vue en coupe selon la ligne C-C de la figure 11B ;
la figure 11D est une vue en coupe selon la ligne D-D de la figure 11B ; et
la figure 12 est une vue en élévation de la variante de réalisation montrée sur la figure 10.

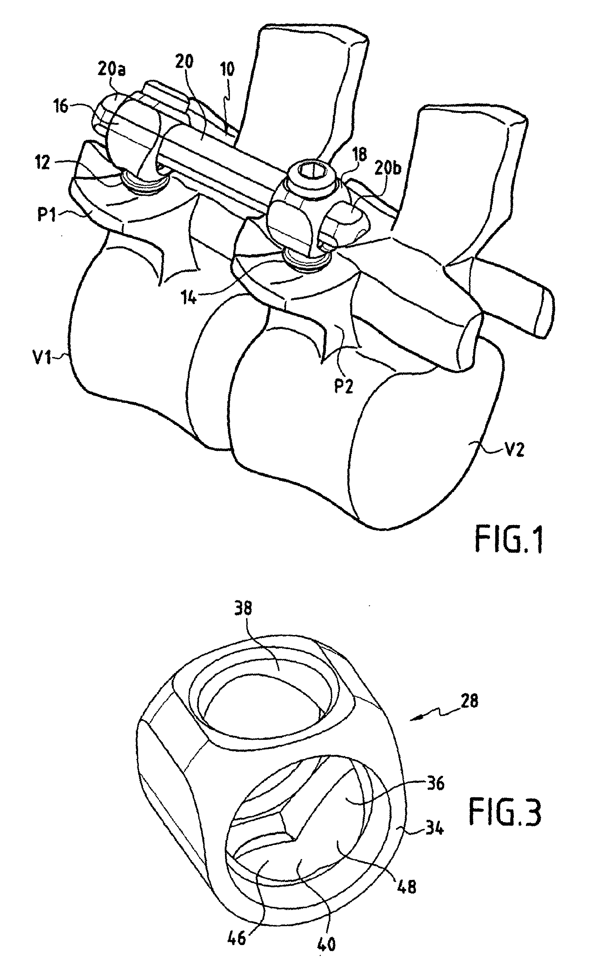

En se référant tout d'abord à la figure 1, on va décrire l'ensemble du dispositif d'immobilisation d'au moins deux vertèbres mis en place.

Sur cette figure, on a représenté une première vertèbre V1 avec son pédicule P1 ainsi qu'une deuxième vertèbre adjacente V2 avec son pédicule P2. Le dispositif d'immobilisation portant la référence générale 10 est constitué par une première vis pédiculaire 12 vissée dans le pédicule P1, une deuxième vis pédiculaire 14 vissée dans le pédicule P2, un premier organe de liaison 16 associé à la vis pédiculaire 12 et un deuxième organe de solidarisation 18 associé à la vis pédiculaire 14. Enfin, le dispositif d'immobilisation 10 comporte une pièce allongée de liaison 20 qui est le plus souvent appelée plaque. Comme le montre cette figure, la pièce de liaison 20 est rendue solidaire par chacune de ses extrémités 20a, 20b des vis pédiculaires 12 et 14 correspondante par un organe de solidarisation 18, 20. On comprend qu'ainsi, après la mise en place des vis 12 et 14, le chirurgien, en réglant la distance entre les organes de solidarisation 16 et 18, peut fixer un écartement entre les vertèbres V1 et V2 à l'aide de la pièce de liaison 20.

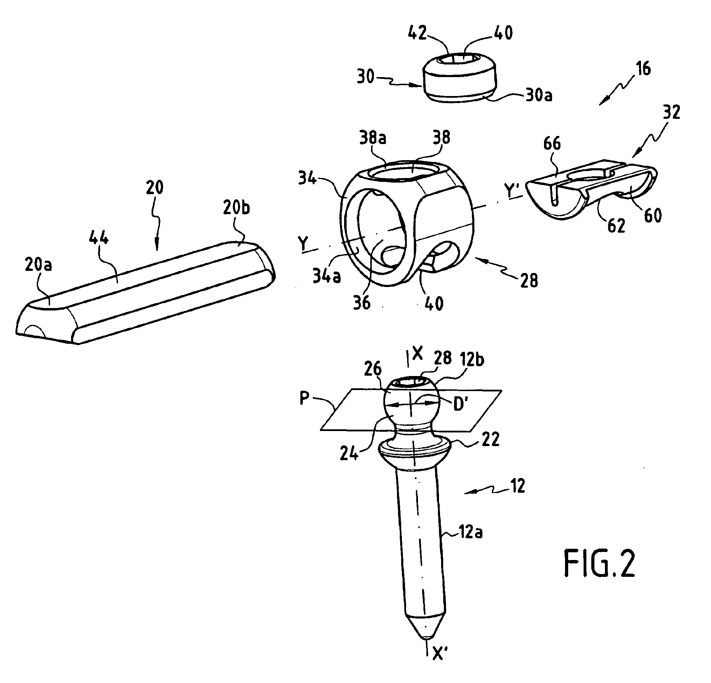

En se référant maintenant à la figure 2, on va décrire plus en détail les différents éléments constitutifs du dispositif d'immobilisation. Sur cette figure, on retrouve la vis pédiculaire 12 qui comporte un corps de vissage 12a et une tête 12b. La vis présente un axe longitudinal X, X' et, de préférence, le corps de vissage 12a est raccordé à la tête 12b par une collerette 22. La tête de vis 12b a la forme d'une partie de surface sphérique qui peut être divisée par un plan P diamétral et perpendiculaire à l'axe X, X' en une première portion de surface sphérique 24 disposée entre le plan P et le corps de vissage 12a et une deuxième portion de surface sphérique 26. La deuxième portion de surface sphérique 26 est limitée par un trou borgne hexagonal ou autre 28 servant à la mise en place de l'outil de vissage. Si l'on considère maintenant l'organe de solidarisation 16, il est constitué essentiellement par une pièce de solidarisation 28, par un organe de serrage 30 et, de préférence, mais non nécessairement, par une pièce intermédiaire 32.

La pièce de solidarisation 28 a la forme générale d'une bague constituée par une paroi 34 dont la face interne 34a est sensiblement cylindrique de révolution autour d'un axe Y, Y'. Cette paroi 34 de la pièce 28 présente un passage axial 36 limité par la face interne 34a et deux ouvertures respectivement référencées 38 et 40 percées dans la paroi 34.

La première ouverture 38 présente une paroi 38a qui est filetée ou qui constitue une partie d'un système à baïonnette. L'ouverture 38 est destinée à recevoir l'organe de serrage 30. Cet organe de serrage comporte une partie filetée ou constituant un système à baïonnette 30a et une tête 40 munie, par exemple, d'un trou borgne 42 pour l'engagement d'un outil de vissage ou de serrage. Sur la figure 2, on a également représenté la pièce allongée de liaison 20 qui présente une première face 44 sensiblement plane destinée à coopérer avec la partie active 30a de l'organe de serrage 30. La pièce 20, ou au moins ses extrémités 20a et 20b, sont définies pour pouvoir être engagées dans le passage axial 36 des pièces de solidarisation 28.

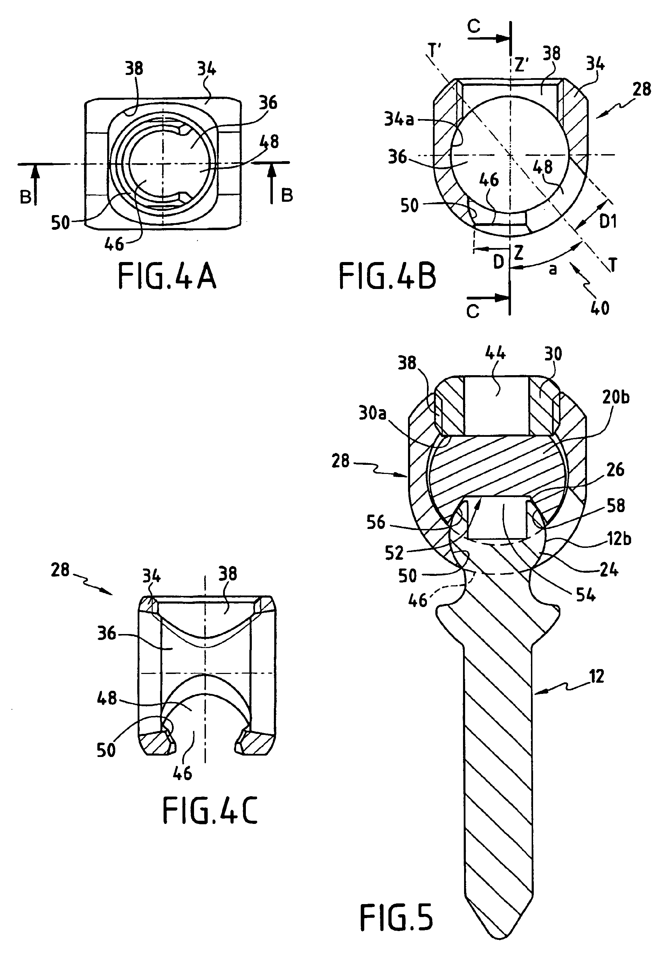

En se référant maintenant aux figures 3 et 4A à 4C, on va décrire plus en détail la pièce de solidarisation 28. Sur la figure 3, on retrouve la paroi 34 de cette pièce avec son passage axial cylindrique 36, son ouverture supérieure 38 destinée à recevoir l'organe de serrage 30 et sa deuxième ouverture ou ouverture inférieure 40. Sur cette figure 3, on voit que la deuxième ouverture 40 est constituée, en fait, par deux parties respectivement référencées 46 et 48 qui communiquent l'une avec l'autre et qui sont angulairement décalées autour de l'axe Y, Y' de la pièce de solidarisation.

Comme le montrent mieux les figures 4A et 4B, la première partie 46 de la deuxième ouverture 40 a la forme d'une portion de cercle (sensiblement un demi-cercle) d'axe Z-Z' confondue avec l'axe de la première ouverture 38. Cette ouverture 46 présente un diamètre D inférieur au diamètre D' de la tête 26 de la vis 12. Le rebord de l'ouverture de la partie 46 de l'ouverture 40 constitue une portée 50, par exemple, en forme de portion de surface sphérique. La deuxième partie d'ouverture 48 a la forme d'une portion de cercle de diamètre D1 et d'axe T, T' angulé d'un angle a par rapport à l'axe Z-Z'. L'angle a peut être égal à 40 degrés. Le diamètre D1 de la deuxième partie 48 de l'ouverture 40 est supérieur au diamètre D' de la tête 26 de la vis 12. Le contour de la deuxième ouverture 40 consiste bien sûr dans l'intersection des portions de cercles correspondant aux parties 46 et 48. On comprend dès à présent que la tête 26 de la vis 12 peut être engagée librement dans le passage axial 36 de la pièce de solidarisation 28 à travers la deuxième partie 48 de l'ouverture 40 lorsque l'axe X, X' de la vis coïncide avec l'axe T, T' de la portion d'ouverture 48. En revanche, si l'on amène l'axe X-X' de la vis 12 en coïncidence avec l'axe Z-Z' de la pièce de solidarisation 28, après avoir introduit sa tête dans le passage axial 36, la première portion de surface sphérique 24 de cette tête vient au contact de la portée sphérique 50, ce qui "emprisonne" la tête de la vis dans le passage axial 36.

Sur la figure 5 on a représenté le montage du dispositif d'immobilisation selon le premier mode de réalisation, c'est-à-dire celui dans lequel l'organe de fixation ne comporte pas la pièce intermédiaire 32. Sur cette figure, on a représenté la vis 12 engagée dans la première partie 46 de la deuxième ouverture 40 de la pièce de solidarisation 28. La première portion de surface sphérique 24 de la tête 12b de la vis est donc en appui sur la portée 50 de cette partie de l'ouverture. Dans ce mode de réalisation, la deuxième face 52 de l'extrémité 20b de la pièce de liaison 20 comporte un évidement longitudinal 54 qui définit deux portées inclinées longitudinales 56 et 58. Lorsque l'extrémité 20b de la pièce de liaison 20 est engagée dans le passage axial 36 de la pièce de solidarisation 28, les portées inclinées 56 et 58 de la pièce 20 sont en regard de la deuxième portion de surface sphérique 26 de la tête 12b de la vis. Lorsque l'on visse l'organe de serrage 30 dans son ouverture taraudée 38, la face active 30a est appliquée contre la face plane 44 de la pièce 20 et l'effort de serrage est transmis par la pièce 20 à la tête 12b de la vis qui est immobilisée en rotation par la coopération des portées 50, 56 et 58. Simultanément, par ce serrage, la pièce 20 est immobilisée en translation par rapport à la pièce de solidarisation 28.

Selon un mode perfectionné de réalisation du dispositif d'immobilisation, chaque organe de solidarisation 16, 18 comporte en outre une pièce intermédiaire 32 qui peut être insérée dans le passage axial 36 de la pièce de liaison 28.

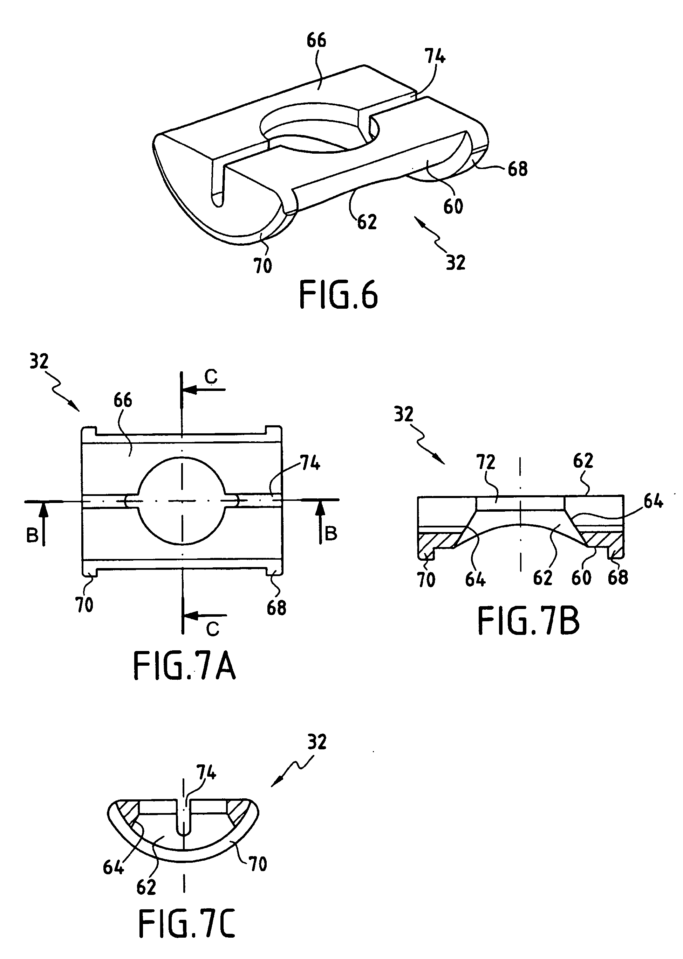

En se référant maintenant aux figures 6 et 7A à 7C on va décrire un premier mode préféré de réalisation de la pièce intermédiaire 32. Celle-ci présente une première face 60 ayant la forme d'une portion de surface cylindrique dont le rayon est sensiblement égal à celui du passage axial 36 de la pièce de solidarisation 28. Dans cette première face 60 est réalisé un évidement 62 qui sera décrit plus en détail ultérieurement. La deuxième face 66 de la pièce 32 est sensiblement plane. De préférence, à chacune de ses extrémités, la pièce 32 est munie d'une nervure 68, 70. Les nervures sont séparées par une longueur axiale L au moins égale à la longueur axiale de la pièce 28.

Comme le montre mieux la figure 7B, l'évidement 62 ménagé dans la pièce 32 comporte une partie active débouchant dans la face 60 qui constitue une portée tronconique 64 ou éventuellement sphérique. De préférence, pour des raisons d'usinage, la partie utile de l'évidement 62 est prolongée par une portion circulaire 72. De préférence également, la pièce 32 comporte une rainure longitudinale 74 disposée selon le plan médian longitudinal de la pièce afin de permettre sa déformation élastique, ainsi qu'on l'expliquera ultérieurement.

La fonction de la pièce intermédiaire 32 est d'être insérée dans le passage axial 36 de la pièce de liaison 28 entre la tête 12b de la vis 12 et l'extrémité 20a de la pièce de liaison 20. Plus précisément, la fonction de cette pièce est de transmettre la force de serrage exercée par l'organe de serrage 30 sur la face supérieure 44 de la pièce de liaison 20 à la deuxième portion sphérique 26 de la tête de la vis 12b par coopération de la portée tronconique 64 de l'évidement 62 de la pièce 32 avec la deuxième portion de surface sphérique de la tête 12b de la vis.

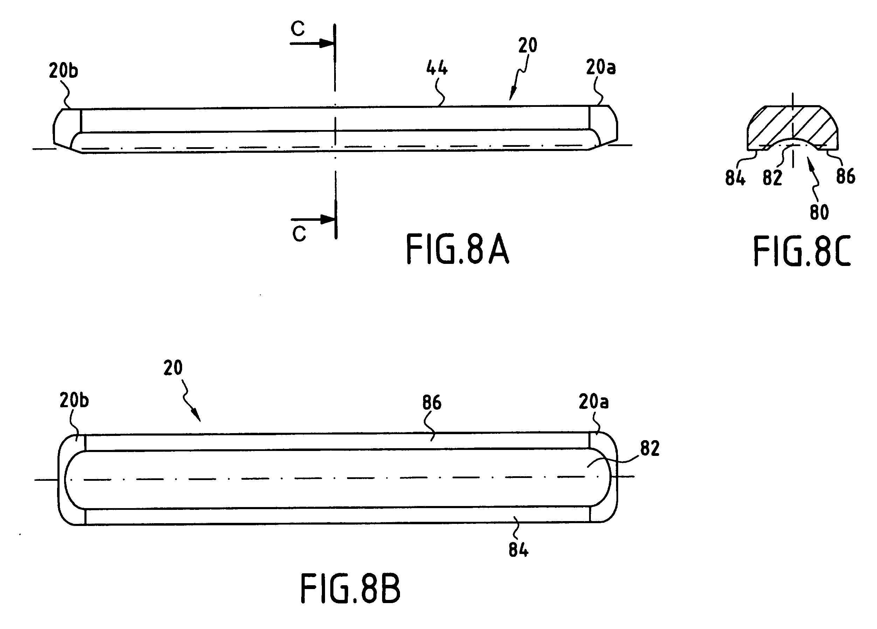

En se référant aux figures 8A, 8B et 8C, on va décrire la forme particulière de la pièce de liaison 20 dans le cas du mode perfectionné de réalisation, la pièce 20 présente toujours une face supérieure plane 44 et sa deuxième face 80 est également plane et comporte un évidement axial 82 de telle manière que cette face 80 définisse deux portées planes longitudinales 84 et 86. Comme on l'expliquera ultérieurement, ces portées 84 et 86 viennent en appui sur la face supérieure plane 66 de la pièce intermédiaire 32.

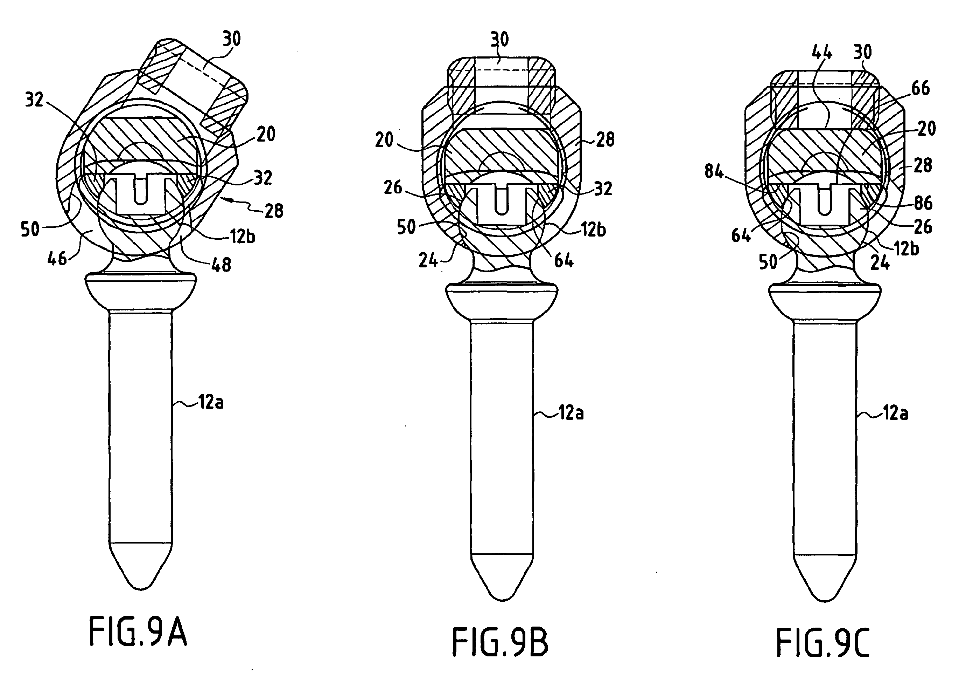

En se référant maintenant aux figures 9A, 9B, 9C, on va décrire l'utilisation et la mise en oeuvre du système d'immobilisation selon son mode de réalisation perfectionné.

Il faut tout d'abord préciser que, de préférence, la pièce intermédiaire 32 est pré-montée à l'intérieur du passage axial 36 de la pièce de solidarisation et que l'organe de serrage 30 est également pré-monté sur l'organe de solidarisation.

Les vis 12 et 14 ayant été vissées par le chirurgien dans les pédicules des vertèbres à immobiliser, celui-ci engage, sur les têtes 12b de ces vis les pièces de solidarisation 28 de telle manière que la deuxième partie 48 de la deuxième ouverture 40 présente un axe T,T' aligné avec celui de la vis 12. La tête 12b de la vis peut être librement engagée par la portion d'ouverture 48 pour pénétrer partiellement dans le passage axial 36. Plus précisément, cette tête 12B vient se loger à l'intérieur de l'évidement 62 de la pièce intermédiaire 32, celle-ci étant dans cet état, libre de pivoter autour de l'axe longitudinal de la pièce de solidarisation. Ensuite, après avoir réalisé cette opération pour les vis 12 et 14, le chirurgien met en place la pièce de liaison allongée 30 ou, plus précisément, les extrémités 20A et 20B de ces pièces ont pu être déjà engagées dans les pièces de solidarisation 28.

Ces opérations ayant été réalisées, le chirurgien provoque le pivotement de la pièce de solidarisation 28 autour de son axe longitudinal de l'angle a afin d'amener l'axe Z,Z' de la première partie 46 de l'ouverture 40 en alignement avec celui de la vis 12. C'est ce qui est représenté sur la figure 9B. Dans cette position, la première portion de surface sphérique 24 de la tête 12B de la vis repose sur la portée 50 de la pièce de solidarisation. Il faut remarquer que, grâce au système rotulant formé par la tête 12B de la vis et par les portées sphériques ou coniques 50 de la pièce 28 et 64 de la pièce intermédiaire, la position de l'élément allongé 20 par rapport aux vis 12 et 14 peut être adaptée angulairement. Une fois que cette opération a été réalisée, il suffit au chirurgien d'activer les organes de serrage 30 des deux pièces de solidarisation 28 pour assurer le blocage et l'immobilisation de la vis 12 de la pièce de liaison 20 et de la pièce due liaison 20 sur l'organe de solidarisation 28.

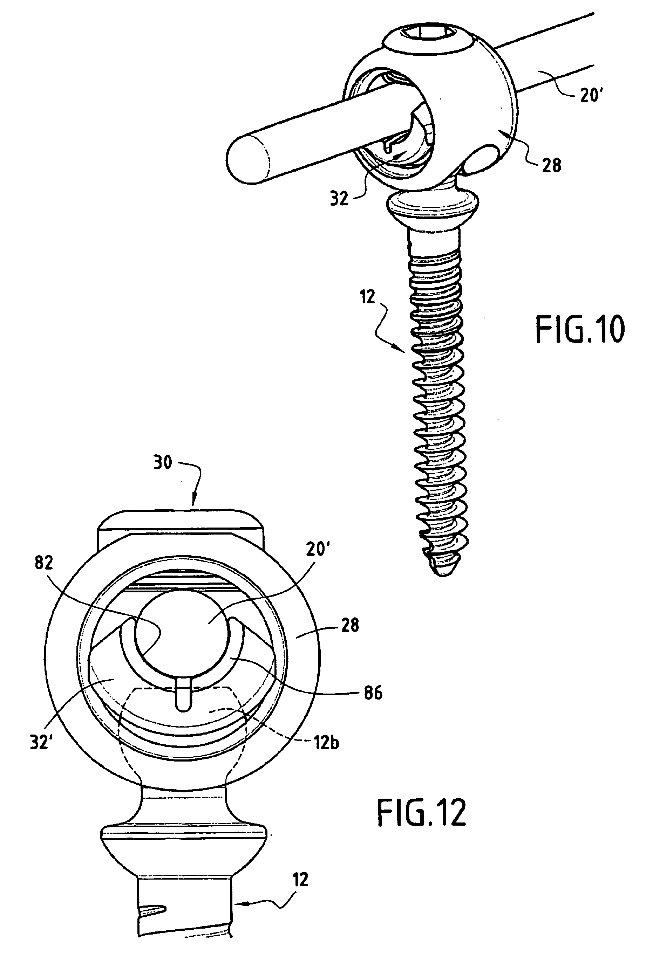

En se référant maintenant aux figures 10 à 12 on va décrire une variante du mode perfectionné de mise en oeuvre de l'invention.

Cette variante se distingue du mode de mise en oeuvre déjà décrit par le fait que l'organe allongé de liaison qui porte la référence 20' sur la figure 10 est une tige à section droite circulaire. On comprend que la pièce intermédiaire qui porte la référence 32' sur la figure 10 doit être modifiée.

En revanche, les vis 12, l'organe de vissage 30 et la pièce de solidarisation 28 ne sont pas modifiés. Ils ne seront donc pas décrits à nouveau.

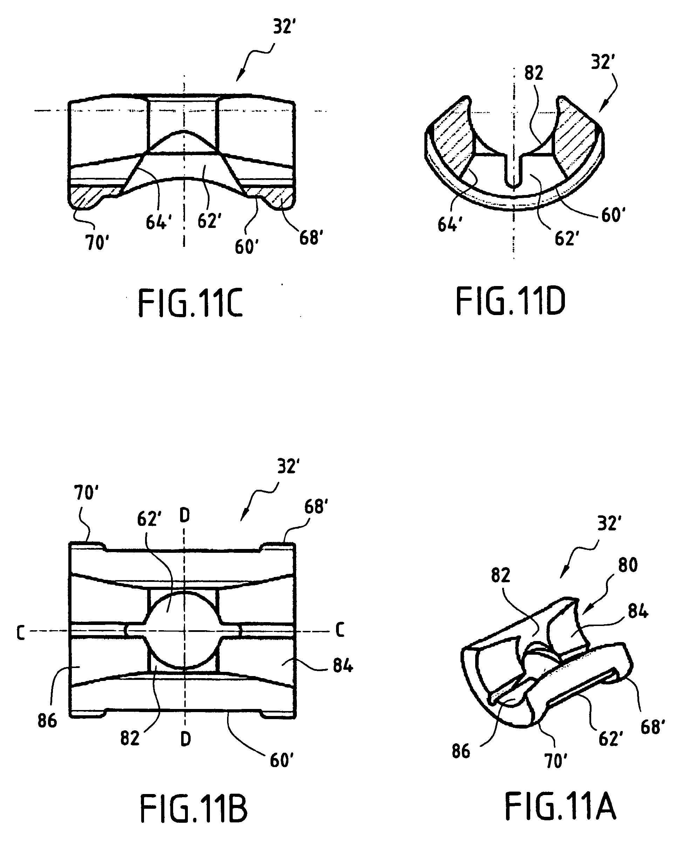

En se référant plus particulièrement aux figures 11A à 11D on va décrire la variante de réalisation de la pièce intermédiaire 32'. La pièce 32' présente une face inférieure 60' en forme de secteur de surface cylindrique destinée à être appliquée contre la paroi interne 34 du passage axial 36 de la pièce de solidarisation 28. La face inférieure 60' se termine à ses extrémités par des nervures 68' et 70' jouant le même rôle que les nervures 68 et 70.

Dans la face inférieure 60' de la pièce 32' est ménagé un évidement 62' qui joue le même rôle que l'évidement 62 et qui comporte une partie tronconique ou éventuellement sphérique 64' destinée à venir en appui sur la tête sphérique 12b de la vis 12.

La face supérieure 80' de la pièce 32' comporte une portée centrale, semi-cylindrique 82, conformée pour recevoir la tige de liaison 20'. La portée centrale 82 se prolonge à ses deux extrémités par des parties semi-tronconiques 84 et 86. L'évidement 62' débouche à son extrémité supérieure dans la portée centrale 82. Cette portée centrale 82 pourrait également être constituée par deux surfaces planes inclinées symétriques par rapport au plan médian de la pièce 32'.

L'utilisation de ce mode de réalisation est sensiblement identique à celui du mode de réalisation illustré sur les figures 9A à 9D. La seule différence réside dans le fait que l'extrémité de la tige de liaison 20' est appliquée sur la portée 82 par l'action de l'organe de vissage 30.

Il résulte de la description précédente du mode de mise en oeuvre du système de solidarisation, que celui-ci permet une mise en place aisée du système tout en permettant l'adaptation nécessaire de la direction de la pièce de liaison par rapport aux vis et en assurant une solidarisation et une immobilisation efficace des vis et de la pièce de liaison.

## Revendications

1. Dispositif d'immobilisation d'au moins deux vertèbres comprenant au moins deux vis (12, 14), une pièce allongée de liaison (20, 20') et au moins deux organes de solidarisation (16, 18), chaque vis (12, 14) comprenant un corps de vissage (12a) et une tête (12b) ayant la forme d'une partie de sphère constituée par une première portion de surface sphérique (24) disposée entre le corps de la vis et un plan diamétral orthogonal à l'axe du corps de la vis et une deuxième portion de surface sphérique (26) ; **caractérisé en ce que**
chaque organe de solidarisation (16, 18) comprend au moins :
une pièce de serrage (30), et
une pièce de solidarisation (28) formée d'une seule pièce ayant la forme d'une bague ayant une paroi latérale (34) entourant un passage axial (36), ladite paroi comportant une première ouverture (38) apte à recevoir ladite pièce de serrage et à coopérer avec elle et une deuxième ouverture (40) comportant une première (46) et une deuxième (48) partie, lesdites deux parties communiquant entre elles et étant angulairement décalées par rapport à l'axe (Y,Y') de la pièce de solidarisation, ladite première partie présentant un axe diamétral (Z, Z') sensiblement confondu avec celui de ladite première ouverture et un rebord (50) formant une portée pour ladite première portion (24) de surface sphérique de la tête de vis, ladite deuxième partie (48) de la deuxième ouverture (40) permettant le libre passage de la tête de la vis, ledit passage axial étant apte à recevoir au moins une extrémité de ladite pièce de liaison (20, 20') et ladite tête de vis (12b), par quoi, la tête de la vis peut être librement introduite dans le passage axial de la pièce de solidarisation, par ladite deuxième partie de la deuxième ouverture, par rotation de ladite pièce de solidarisation, la portée (50) de la première partie de la deuxième ouverture est amenée en regard de la première portion de surface sphérique de la tête de vis, et, par activation de la pièce de serrage, l'extrémité de la pièce de liaison et la tête de la vis sont immobilisées en rotation et en translation par rapport à ladite pièce de solidarisation.

2. Dispositif d'immobilisation selon la revendication 1, **caractérisé en ce que** ledit organe de solidarisation (16, 18) comprend en outre une pièce intermédiaire (32, 32') insérable dans le passage axial (36) de la pièce de solidarisation, ladite pièce intermédiaire (32, 32') présentant une première face (60) apte à être mise en regard de la face interne de la paroi (34) de la pièce de solidarisation (28), ladite pièce intermédiaire présentant un évidement, débouchant dans ladite première face, formant une portée pour au moins une partie de ladite deuxième portion (26) de surface sphérique de la tête de vis (12b), et une deuxième face (66) d'appui apte à coopérer avec l'extrémité (20a) de la pièce de liaison (20, 20'), par quoi, lorsque ladite pièce intermédiaire (32) est insérée dans le passage axial (36) de la pièce de liaison, la force de serrage développée par l'organe de serrage (30) est transmise à ladite pièce intermédiaire par l'intermédiaire de l'extrémité de la pièce de liaison.

3. Dispositif d'immobilisation selon la revendication 1, **caractérisé en ce qu'**au moins chaque extrémité (20a, 20b) de ladite pièce de liaison (20) comporte une première face (44) sensiblement plane et une deuxième face (52) présentant un évidement longitudinal (54) définissant deux portées inclinées (56, 58) aptes à coopérer avec la deuxième portion (26) de surface sphérique de la tête (12b) de vis.

4. Dispositif d'immobilisation selon la revendication 2, **caractérisé en ce que** ladite pièce intermédiaire (32, 32') présente à chacune de ses extrémités une nervure (68, 70) faisant saillie hors de sa première face (60) pour coopérer avec les faces d'extrémité de ladite pièce de solidarisation (28) lorsque la pièce intermédiaire (32, 32') est engagée dans le passage axial (36) de la pièce de solidarisation (28).

5. Dispositif d'immobilisation selon l'une quelconque des revendications 2 et 4, **caractérisé en ce qu'**au moins chaque extrémité (20a, 20b) de la pièce de liaison (20) présente une première face sensiblement plane (44) pour coopérer avec l'organe de serrage (30) et une deuxième face (80) définissant deux portées sensiblement plane (84, 86) pour coopérer avec la deuxième face (66) de ladite pièce intermédiaire (32).

6. Dispositif d'immobilisation selon une quelconque des revendications 2 et 4 **caractérisé en ce que** ladite pièce de liaison (20') a une section droite circulaire et **en ce que** ladite deuxième face de la pièce intermédiaire (32') présente une portée à section droite en forme d'arc de cercle apte à recevoir une extrémité de ladite pièce de liaison (20').

## Claims

1. A system for immobilizing two or more vertebrae, which system comprises two or more screws (12, 14), an elongate connecting member (20, 20') and two or more fastening systems (16, 18), each screw (12, 14) comprising a screw body (12a) and a screw head (12b) having the shape of a portion of a sphere consisting of a first spherical surface portion (24) between the screw body and a diametral plane orthogonal to the axis of the screw body and a second spherical surface portion (26); **characterized in that**
each fastening system (16, 18) comprises at least:
- a clamping member (30); and
- a fastening member (28) formed in one piece having the shape of a ring having a lateral wall (34) around an axial passage (36), said wall including a first aperture (38) adapted to receive and to cooperate with said clamping member and a second aperture (40) having a first portion (46) and a second portion (48), said two portions communicating with each other and being angularly offset relative to the axis (Y, Y') of the fastening member, said first portion having a diametral axis (Z, Z') substantially coinciding with that of said first aperture and a rim (50) forming a bearing surface for said first spherical surface portion (24) of the screw head, said second portion (48) of the second aperture (40) allowing the screw head to pass through it, said axial passage being adapted to receive at least one end of said connecting member (20, 20') and said screw head (12b), whereby the screw head may be freely introduced into the axial passage of the fastening member via said second portion of the second aperture by rotating said fastening member, the bearing surface (50) of the first portion of the second aperture is made to face the first spherical surface portion of the screw head and, by activation of the clamping member, the end of the connecting member and the screw head are immobilized against rotation and against movement in translation relative to said fastening member.

2. An immobilization system according to claim 1, **characterized in that** said fastening system (16, 18) further comprises an intermediate member (32, 32') adapted to be inserted into the axial passage (36) of the fastening member and having a first face (60) adapted to be made to face the internal face of the wall (34) of the fastening member (28), a recess opening onto said first face, forming a bearing surface for at least a portion of said second spherical surface portion (26) of the screw head (12b), and a second bearing face (66) adapted to cooperate with the ends (20a) of the connecting member (20, 20') whereby, when said intermediate member (32) is inserted into the axial passage (36) of the connecting member, the clamping force produced by the clamping member (30) is transmitted to said intermediate member via the end of the connecting member.

3. An immobilization system according to claim 1, **characterized in that** each end (20a, 20b) of said connecting member (20) has a substantially plane first face (44) and a second face (52) including a longitudinal recess (54) defining two inclined bearing surfaces (56, 58) adapted to cooperate with the second spherical surface portion (26) of the screw head (12b).

4. An immobilization system according to claim 2,
**characterized in that** said intermediate member (32, 32') has at each end a rib (68, 70) projecting from its first face (60) to cooperate with the end faces of said fastening member (28) when the intermediate member (32, 32') is engaged in the axial passage (36) of the fastening member (28).

5. An immobilization system according to claim 2 or claim 4, **characterized in that** each end (20a, 20b) of the connecting member (20) has a substantially plane first face (44) for cooperating with the clamping member (30) and a second face (80) defining two substantially plane bearing surfaces (84, 86) for cooperating with the second face (66) of said intermediate member (32).

6. An immobilization system according to claim 2 or claim 4, **characterized in that** said connecting member (20') has a circular cross-section and **in that** said second face of the intermediate member (32') includes a bearing surface that has a cross-section in the shape of a circular arc adapted to receive an end of said connecting member (20').

## Patentansprüche

1. Vorrichtung zum Festlegen von wenigstens zwei Wirbeln, mit wenigstens zwei Schrauben (12, 14), einem länglichen Verbindungsteil (20, 20') sowie wenigstens zwei Organen zum festen Verbinden (16, 18), wobei jede Schraube (12, 14) einen Einschraubkörper (12a) und einen Kopf (12b) umfaßt, welcher die Form eines Kugelteils aufweist, das von einem ersten Kugelflächenabschnitt (24), der zwischen dem Körper der Schraube und einer zur Achse des Körpers der Schraube orthogonalen Diametralebene angeordnet ist, sowie von einem zweiten Kugelflächenabschnitt (26) gebildet ist, **dadurch gekennzeichnet, daß** jedes Organ zum festen Verbinden (16, 18) wenigstens folgendes umfaßt:
ein Klemmteil (30) und
ein einstückig ausgebildetes Teil zum festen Verbinden (28), das die Form eines Rings aufweist, der eine Seitenwand (34) hat, welche einen axialen Durchgang (36) umgibt, wobei die Wand eine erste Öffnung (38), welche geeignet ist, das Klemmteil aufzunehmen und mit diesem zusammenzuwirken, sowie eine zweite Öffnung (40) mit einem ersten (46) und einem zweiten Teil (48) aufweist, wobei die beiden Teile miteinander verbunden sind und gegenüber der Achse (Y, Y') des Teils zum festen Verbinden winkelmäßig versetzt sind, wobei der erste Teil eine diametrale Achse (Z, Z'), die im wesentlichen mit derjenigen der ersten Öffnung zusammenfällt, sowie einen eine Auflagefläche für den ersten Kugelflächenabschnitt (24) des Schraubenkopfes bildenden Rand (50) aufweist, wobei der zweite Teil (48) der zweiten Öffnung (40) den freien Durchgang des Kopfes der Schraube ermöglicht, wobei der axiale Durchgang geeignet ist, wenigstens ein Ende des Verbindungsteils (20, 20') und den Schraubenkopf (12b) aufzunehmen, wodurch der Kopf der Schraube über den zweiten Teil der zweiten Öffnung, durch Drehen des Teils zum festen Verbinden in den axialen Durchgang des Teils zum festen Verbinden frei einführbar ist, die Auflagefläche (50) des ersten Teils der zweiten Öffnung gegenüber dem ersten Kugelflächenabschnitt des Schraubenkopfes plaziert wird und, durch Aktivieren des Klemmteils, das Ende des Verbindungsteils und der Kopf der Schraube gegenüber dem Teil zum festen Verbinden gegen ein Drehen und Verschieben festgelegt werden.

2. Festlegungsvorrichtung nach Anspruch 1, **dadurch gekennzeichnet, daß** das Organ zum festen Verbinden (16, 18) ferner ein Zwischenteil (32, 32') aufweist, das in den axialen Durchgang (36) des Teils zum festen Verbinden eingefügt werden kann, wobei das Zwischenteil (32, 32') eine erste Fläche (60), die geeignet ist, gegenüber der Innenseite der Wand (34) des Teils zum festen Verbinden (28) plaziert zu werden, wobei das Zwischenteil eine in die erste Fläche mündende Ausnehmung aufweist, die eine Auflagefläche für wenigstens einen Teil des zweiten Kugelflächenabschnitts (26) des Schraubenkopfes (12b) bildet, sowie eine zweite Auflagefläche (66) aufweist, die geeignet ist, mit dem Ende (20a) des Verbindungsteils (20, 20') zusammenzuwirken, wodurch dann, wenn das Zwischenteil (32) in den axialen Durchgang (36) des Verbindungsteils eingefügt ist, die durch das Klemmorgan (30) aufgebrachte Klemmkraft über das Ende des Verbindungsteils auf das Zwischenteil übertragen wird.

3. Festlegungsvorrichtung nach Anspruch 1, **dadurch gekennzeichnet, daß** wenigstens jedes Ende (20a, 20b) des Verbindungsteils (20) eine im wesentlichen ebene erste Fläche (44) sowie eine zweite Fläche (52) umfaßt, die eine Längsausnehmung (54) aufweist, welche zwei geneigte Auflageflächen (56, 58) definiert, die geeignet sind, mit dem zweiten Kugelflächenabschnitt (26) des Schraubenkopfes (12b) zusammenzuwirken.

4. Festlegungsvorrichtung nach Anspruch 2, **dadurch gekennzeichnet, daß** das Zwischenteil (32, 32') an jedem seiner Enden eine Rippe (68, 70) aufweist, die aus der ersten Fläche (60) hervorragt, um mit den Endflächen des Teils zum festen Verbinden (28) zusammenzuwirken, wenn das Zwischenteil (32, 32') in den axialen Durchgang (36) des Teils zum festen Verbinden (28) eingesteckt ist.

5. Festlegungsvorrichtung nach einem der Ansprüche 2 und 4, **dadurch gekennzeichnet, daß** wenigstens jedes Ende (20a, 20b) des Verbindungsteils (20) eine im wesentlichen ebene erste Fläche (44), um mit dem Klemmorgan (30) zusammenzuwirken, sowie eine zweite Fläche (80) aufweist, die zwei im wesentlichen ebene Auflageflächen (84, 86) definiert, um mit der zweiten Fläche (66) des Zwischenteils (32) zusammenzuwirken.

6. Festlegungsvorrichtung nach einem der Ansprüche 2 und 4, **dadurch gekennzeichnet, daß** das Verbindungsteil (20') einen kreisförmigen Querschnitt aufweist, und daß die zweite Fläche des Zwischenteils (32') eine Auflagefläche mit kreisbogenförmigem Querschnitt aufweist, die geeignet ist, ein Ende des Verbindungsteils (20') aufzunehmen.
